# EUROPÄISCHE PATENTANMELDUNG

(11) **EP 3 563 786 A1**
(43) Veröffentlichungstag der Anmeldung: **06.11.2019**
(21) Anmeldenummer: 18170762.1
(22) Anmeldetag: 04.05.2018
(51) Int. Cl.: A61B 18/14

(54) **KATHETERKOPF, KATHETER UND HERSTELLUNGSVERFAHREN**

(71) Anmelder: VascoMed GmbH, 79589 Binzen (DE)
(72) Erfinder: Bitzer, Andreas, 8032 Zürich (CH)
(74) Vertreter: Engelhardt, Björn

(57) **Zusammenfassung**

Die Offenbarung betrifft einen Katheterkopf (1) mit mehreren Spülöffnungen (2a, 2b), die mit einer Spülzuleitung (3) verbunden sind, und einem Sensor (4). Der Sensor (4) ist innerhalb des Katheterkopfes (1) angeordnet. Weiterhin sind ein Katheter und ein Herstellungsverfahren offenbart.

## Beschreibung

Die Offenbarung betrifft einen Katheterkopf, einen Katheter und ein Herstellungsverfahren für einen Katheter.

### Hintergrund

Es sind verschiedene Kathetertypen bekannt, beispielsweise Ablationskatheter und Diagnosekatheter.

Ein Beispiel für einen gespülten Ablationskatheter ist im Dokument EP 2 380 517 A1 offenbart. In der Ablationselektrode des Katheters sind gewinkelte Spülkanäle gebildet. Der Katheter kann darüber hinaus einen Temperatursensor aufweisen.

Ein anderer gespülter Katheter ist im Dokument US 2017/0258522 A1 beschrieben. Hier ist vorgesehen, einen Temperatursensor außerhalb der Elektrode oder von Spülöffnungen entfernt anzuordnen.

Das Dokument US 2017/0215802 A1 offenbart einen gespülten Ablationskatheter mit Kraftmessung und Positionsbestimmung. Ein Positionssensor ist hinter dem Katheterkopf in einem deflektierbaren Bereich des Katheters angeordnet. Hier wirken beispielsweise Biegemomente auf den Sensor. Das kann den Sensor beschädigen oder die Sensitivitätseigenschaften stören.

Ein weiterer Ablationskatheter ist in dem Dokument US 9,131,982 B2 beschrieben.

### Zusammenfassung

Aufgabe ist es, verbesserte Technologien für einen Katheter anzugeben. Insbesondere soll der Aufbau des Katheters vereinfacht werden.

Es sind ein Katheterkopf nach Anspruch 1, ein Katheter nach Anspruch 12 sowie ein Verfahren nach Anspruch 13 offenbart. Weitere Ausführungsformen sind Gegenstand von abhängigen Ansprüchen.

Nach einem Aspekt ist ein Katheterkopf bereitgestellt. Der Katheterkopf weist mehrere Spülöffnungen auf, die mit einer Spülzuleitung verbunden sind. Des Weiteren weist der Katheterkopf einen Sensor auf. Der Sensor ist innerhalb des Katheterkopfes angeordnet.

Gemäß einem weiteren Aspekt ist ein Katheter mit einem Katheterkopf entsprechend der vorliegenden Offenbarung bereitgestellt. Der Katheter kann beispielsweise ein Ablationskatheter oder ein Diagnosekatheter (Mapping-Katheter) sein.

Ein anderer Aspekt betrifft ein Verfahren zum Herstellen eines Katheters. Das Verfahren umfasst die Schritte: Bereitstellen eines Katheterkörpers und Befestigen (z. B. mittels Kleben oder Schweißen) eines Katheterkopfes entsprechend der vorliegenden Offenbarung an einem distalen Ende des Katheterkörpers.

Aus dem Stand der Technik sind Katheter mit Sensoren bekannt, die hinter dem Katheterkopf angeordnet sind. Gemäß der vorliegenden Offenbarung ist vorgesehen, den Sensor in den Katheterkopf zu integrieren. Die Integration der Spülkanalanordnung und des Sensors im Katheterkopf ermöglicht eine äußerst kompakte Bauweise.

Die mehreren Spülöffnungen können derart am Katheterkopf angeordnet sein, dass sie ein Volumen aufspannen. Beispielsweise können einige der mehreren Spülöffnungen an einer Seitenwand des Katheterkopfs angeordnet sein. Wenigstens eine Spülöffnung (oder einige der mehreren Spülöffnungen) kann an einem distalen Ende des Katheterkopfs (der Katheterspitze) angeordnet sein. Die Spülöffnungen an der Seitenwand und an der Spitze des Katheters können als Punkte angesehen werden, die ein Volumen aufspannen, wenn sie miteinander verbunden werden. Der Sensor kann in dem von den Spülöffnungen aufgespannten Volumen angeordnet sein. Der Sensor kommt jedoch nicht mit der Spülflüssigkeit in Kontakt, sondern ist hiervon getrennt. Beispielsweise kann der Sensor in einem Sensorlumen angeordnet sein.

Der Katheterkopf und/oder der Katheter können einen Durchmesser von bis zu 7 Fr (French, auch Charriere genannt, 1 Fr = 1/3 mm) haben.

Der Katheterkopf kann als Modul gebildet sein. Katheter werden vom Kopf (distales Ende) her aufgebaut. Der Katheterkopf stellt daher das erste Bauteil in der Fertigungskette dar. Der Katheterkopf mit der Spülkanalanordnung und dem Sensor kann ein Katheterkopfmodul bilden, das an verschiedenen Katheterkörpern befestigt werden kann, um unterschiedliche Katheter zu bauen.

An dem Katheterkopf kann eine Elektrode gebildet sein. Die Elektrode kann ein Metall (z. B. Gold) oder eine Metalllegierung enthalten. Alternativ kann die Elektrode aus einem Metall (z. B. Gold) oder einer Metalllegierung bestehen. Die mehreren Spülöffnungen können in der Elektrode gebildet sein.

Der Sensor kann als Positionssensor oder als Kraftsensor ausgebildet sein. Ein Positionssensor ermöglicht es, die Position des Katheterkopfs zu bestimmen. Dies erleichtert die Orientierung während eines Eingriffs mit dem Katheter.

Der Sensor kann eine Spule sein. Die Spule kann einen Durchmesser von 0,45 mm und eine Länge von 5 - 6 mm aufweisen. An der Spule können zwei Kupferleitungen (z. B. mit einem Durchmesser von 0,1 mm) angeordnet sein, um die gemessenen Signale nach außen zu übertragen, z. B. zu einem proximalen Ende des Katheters. Der Sensor kann als fünfdimensionaler (5D) oder sechsdimensionaler (6D) Positionssensor ausgebildet sein. Ein 6D-Sensor kann zwei Spulen umfassen, die beide in dem Katheterkopf angeordnet sind.

Der Sensor kann ein Magnetfeld-Sensor sein. Der Magnetfeld-Sensor kann im Niederfrequenz-Bereich arbeiten und durch ein Metallgehäuse, wie beispielsweise eine Katheterspitze, Signale empfangen.

Der Sensor kann in einem Sensorlumen angeordnet sein, wobei das Sensorlumen von der Spülzuleitung separat gebildet ist. Das Sensorlumen und die Spülzuleitung können nebeneinander angeordnet sein.

Der Sensor kann parallel zur Längsachse des Katheterkopfes angeordnet sein, z. B. in dem Sensorlumen.

Es kann vorgesehen sein, dass einige der mehreren Spülöffnungen in einer Ebene entlang des Umfangs des Katheterkopfes angeordnet sind.

In einer Ausführungsform kann vorgesehen sein, dass wenigstens eine der mehreren Spülöffnungen an einem distalen Ende des Katheterkopfes angeordnet ist.

Der Katheterkopf kann ein Kernelement und eine Kappe aufweisen, wobei in dem Kernelement die Spülzuleitung und der Sensor angeordnet sind, wobei in der Kappe mehrere Spülöffnungen gebildet sind und wobei die Kappe an dem Kernelement befestigt ist.

Ein Zwischenbereich kann zwischen dem Kernelement und der Kappe gebildet sein. Der Zwischenbereich bildet eine Verbindung zwischen der Spülzuleitung und den Spülöffnungen.

In einer Ausführungsform weist der Katheterkopf eine Ablationselektrode auf, in welcher mehrere Spülöffnungen gebildet sind. Die mehreren Spülöffnungen sind mit einer Spülzuleitung im Inneren des Katheterkopfes verbunden. Des Weiteren ist ein Sensor (z. B. ein Magnetfeld-Sensor zur Positionsbestimmung) in dem Katheterkopf angeordnet. Die Merkmale, welche im Zusammenhang mit dem Katheterkopf und dem Katheter offenbart sind, können in analoger Weise auf das Verfahren angewendet werden und umgekehrt.

### Beschreibung von Ausführunsgbeispielen

Im Folgenden werden beispielhafte Ausführungsformen unter Bezugnahme auf Figuren näher erläutert. Es zeigen:
- Fig. 1: eine schematische Darstellung einer ersten Ausführungsform eines Katheterkopfes,
- Fig. 2: ein Querschnitt des Katheterkopfes nach Fig. 1,
- Fig. 3: eine schematische Seitenansicht einer zweiten Ausführungsform eines Katheterkopfes in einem nicht montierten Zustand und
- Fig. 4: eine schematische Seitenansicht des Katheterkopfes nach Fig. 3 in einem montierten Zustand.

Im Folgenden werden für gleiche Komponenten gleiche Bezugszeichen verwendet.

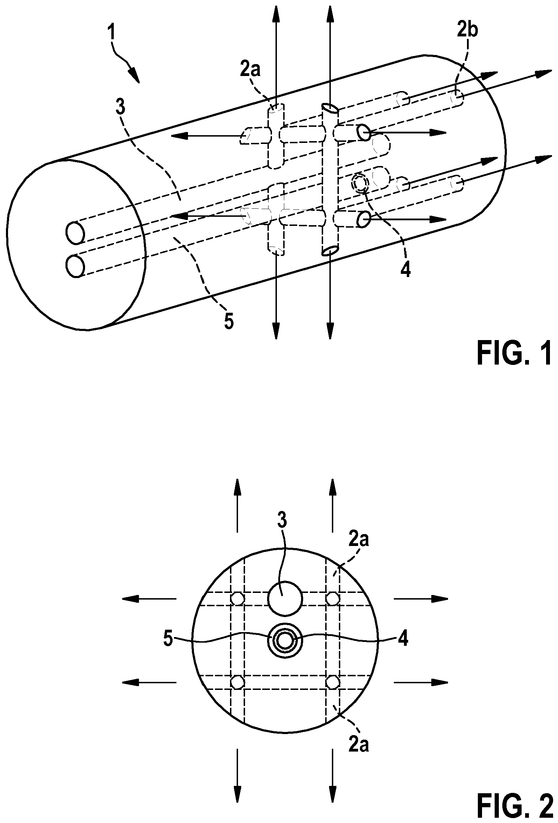

Fig. 1 zeigt eine schematische Darstellung eines Katheterkopfs 1 mit seitlichen Spülöffnungen 2a und frontalen Spülöffnungen 2b (an einem distalen Ende des Katheterkopfes). Die seitlichen Spülöffnungen 2a sind in der gezeigten Ausführungsform mit einer rautenförmigen Spülkanalanordnung gebildet. Sowohl die seitlichen Spülöffnungen als auch die frontalen Spülöffnungen 2b sind mit einer Spülzuleitung 3 verbunden. Mit der Spülzuleitung 3 wird eine Spülflüssigkeit von einem proximalen Ende zugeführt und über die seitlichen und frontalen Spülöffnungen 2a, 2b abgegeben.

Neben der Spülzuleitung 3 ist ein Sensorlumen 5 gebildet. In dem Sensorlumen 5 ist ein Sensor 4 angeordnet. Der Sensor 4 ist hierbei in einem Volumen angeordnet, dass von den seitlichen und frontalen Spülöffnungen 2a, 2b aufgespannt wird. Somit befindet sich der Sensor 4 sehr nahe an dem distalen Ende des Katheterkopfs 1.

Der Sensor 4 kann beispielsweise ein Magnetfeld-Sensor zur Positionsbestimmung des Katheterkopfs sein. In diesem Fall kann die Position der Spitze des Katheters sehr genau bestimmt werden.

Der Katheterkopf 1 ist als einteiliges Bauteil ausgeführt. Ein derartiger Katheterkopf kann in einem CNC-Prozessschritt (CNC - Computerized Numerical Control, Verfahren zur Steuerung von Werkzeugmaschinen) hergestellt werden, was einfach und kostengünstig ist oder mittels eines additiven Fertigungsverfahrens wie z. B. Lasersintern.

Ein Querschnitt des Katheterkopfs 1 nach Fig. 1 ist in Fig. 2 dargestellt. Die Spülzuleitung 3 ist exzentrisch zum Mittelpunkt des Katheterkopfs angeordnet.

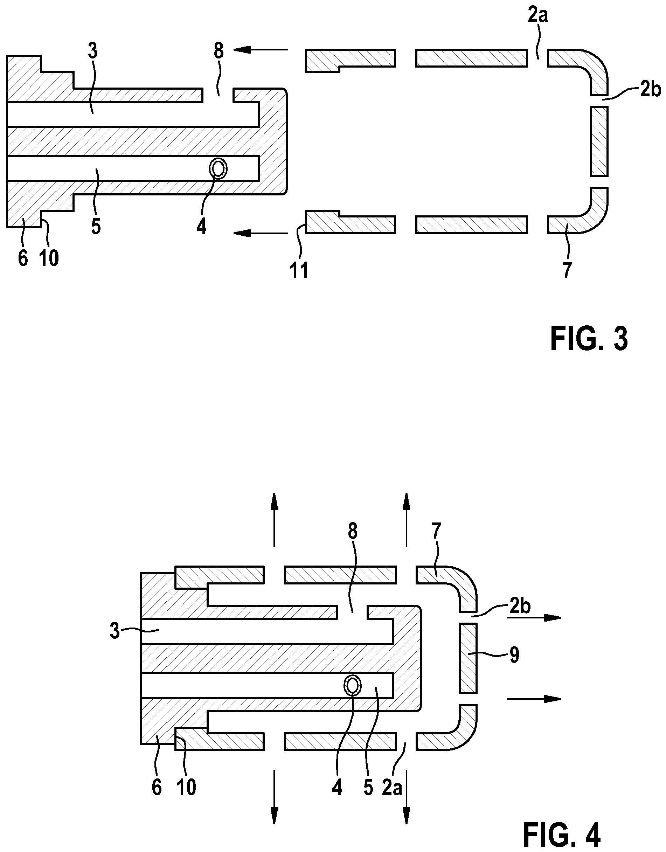

Fig. 3 zeigt einen zweiteiligen Katheterkopf mit einem Kernelement 6 und einer Kappe 7. In der Kappe 7 sind die seitlichen Spülöffnungen 2a und die frontalen Spülöffnungen 2b gebildet. Die Spülzuleitung 3 weist eine Austrittsöffnung 8 auf, welche seitlich aus der Spülzuleitung heraus geführt ist. Zur Montage wird die Kappe 7 auf das Kernelement 6 geführt, bis ein proximales Ende 11 der Kappe 7 an einer Kante 10 anschlägt. Dann wird die Kappe 7 am Kernelement 6 befestigt, z. B. verschweißt oder verklebt.

Im montierten Zustand ist ein Zwischenraum 9 zwischen dem Kernelement 6 und der Kappe 7 gebildet, in welchem ein gleichmäßiger Druck der Spülflüssigkeit aufgebaut wird.

Die Kappe 7 kann als Elektrode eines Katheters wirken. Hierzu kann die Kappe 7 aus einem Metall (z. B. Gold) oder einer Metalllegierung bestehen.

Ein Katheter mit einem Katheterkopf gemäß der vorliegenden Offenbarung kann folgende Vorteile haben:
- kompakte Bauweise,
- vereinfachte Katheterkonstruktion,
- guter Schutz für den Sensor, da dieser nicht im deflektierbaren Bereich des Katheters angeordnet ist, und
- hohe Genauigkeit, z. B. bezüglich der Position, da der Sensor am relevanten Ort (nahe der Katheterspitze) angeordnet ist.

Die in der Beschreibung, den Ansprüchen und den Figuren offenbarten Merkmale können für die Verwirklichung von Ausführungsformen sowohl einzeln als auch in beliebiger Kombination miteinander relevant sein.

### Bezugszeichenliste:

- 1: Katheterkopf
- 2a: seitliche Spülöffnung
- 2b: frontale Spülöffnung
- 3: Spülzuleitung
- 4: Sensor
- 5: Sensorlumen
- 6: Kernelement
- 7: Kappe
- 8: Austrittsöffnung der Spülzuleitung
- 9: Zwischenraum
- 10: Kante des Kernelements
- 11: proximales Ende der Kappe

## Patentansprüche

1. Katheterkopf (1) mit
- mehreren Spülöffnungen (2a, 2b), die mit einer Spülzuleitung (3) verbunden sind, und
- einem Sensor (4),
wobei der Sensor (4) innerhalb des Katheterkopfes (1) angeordnet ist.

2. Katheterkopf (1) nach Anspruch 1, wobei die mehreren Spülöffnungen derart am Katheterkopf angeordnet sind, dass sie ein Volumen aufspannen, und wobei der Sensor in dem von den mehreren Spülöffnungen aufgespannten Volumen angeordnet ist.

3. Katheterkopf (1) nach Anspruch 1 oder 2, wobei der Katheterkopf (1) als Modul gebildet ist.

4. Katheterkopf (1) nach einem der vorangehenden Ansprüche, wobei der Sensor (4) als Positionssensor oder als Kraftsensor ausgebildet ist.

5. Katheterkopf (1) nach einem der vorangehenden Ansprüche, wobei der Sensor (4) eine Spule ist.

6. Katheterkopf (1) nach einem der vorangehenden Ansprüche, wobei der Sensor (4) in einem Sensorlumen (5) angeordnet ist und wobei das Sensorlumen (5) von der Spülzuleitung (3) separat gebildet ist.

7. Katheterkopf (1) nach Anspruch 6, wobei das Sensorlumen (5) und die Spülzuleitung (3) nebeneinander angeordnet sind.

8. Katheterkopf (1) nach einem der vorangehenden Ansprüche, wobei einige der mehreren Spülöffnungen (2a) in einer Ebene entlang des Umfangs des Katheterkopfes angeordnet sind.

9. Katheterkopf (1) nach einem der vorangehenden Ansprüche, wobei wenigstens eine der mehreren Spülöffnungen (2b) an einem distalen Ende des Katheterkopfes (1) angeordnet ist.

10. Katheterkopf (1) nach einem der vorangehenden Ansprüche, aufweisend ein Kernelement (6) und eine Kappe (7), wobei in dem Kernelement (6) die Spülzuleitung (3) und der Sensor (4) angeordnet sind, wobei in der Kappe (7) mehrere Spülöffnungen (2a, 2b) gebildet sind und wobei die Kappe (7) an dem Kernelement (6) befestigt ist.

11. Katheterkopf (1) nach Anspruch 10, wobei ein Zwischenbereich (9) zwischen dem Kernelement (6) und der Kappe (7) gebildet ist, welcher eine Verbindung zwischen der Spülzuleitung (3) und den mehreren Spülöffnungen (2a, 2b) bildet.

12. Katheter mit einem Katheterkopf (1) nach einem der vorangehenden Ansprüche.

13. Verfahren zum Herstellen eines Katheters, wobei das Verfahren folgende Schritte umfasst:
- Bereitstellen eines Katheterkörpers und
- Befestigen eines Katheterkopfes (1) nach einem der vorangehenden Ansprüche an einem distalen Ende des Katheterkörpers.
